Europäisches Patentamt

European Patent Office

Office européen des brevets

(11) Publication number: **0 582 736 A1**

# EUROPEAN PATENT APPLICATION

(21) Application number: **92113700.6**

(22) Date of filing: **11.08.92**

(51) Int. Cl.⁵: **G01N 33/50**, //G01N15/14

(43) Date of publication of application:
**16.02.94 Bulletin 94/07**

(84) Designated Contracting States:
**AT BE CH DE ES FR GB IT LI NL**

(71) Applicant: **ABBOTT LABORATORIES**
**One Abbott Park Road**
**Abbott Park, Illinois 60064-3500(US)**

(72) Inventor: **Wong, Show-Chu**
**903 Bainbridge Court**
**Sunnyvale, California 94087(US)**

(74) Representative: **Modiano, Guido, Dr.-Ing. et al**
**Modiano & Associati S.r.l.**
**Via Meravigli, 16**
**I-20123 Milano (IT)**

(54) Flow cytometry sheath reagent for elimination of red cells with preservation of nucleated cells.

㊼ A reagent is described which when mixed with whole blood, lyses the red cells substantially instantly, but does not substantially affect the optical, light scattering properties of the white blood cells. This reagent enables the performance of a five part leukocyte differential by flow cytometry and light scattering in a single sample path white cell analyzer. In its preferred embodiment, the lytic agent combines the functions of leukoprotective and antimicrobial agents, selected nonionic surfactants, a lytic and wetting agent and an appropriate buffer to provide pH buffering and to increase the solution's conductivity and red blood cell lysing capability.

## Technical Field

This invention relates generally to reagents and methods useful in flow cytometry measurement of cell populations and more specifically, to the use of a sheath reagent containing selected components acting as a red blood cell lyse and flow cytometry sheath. This new reagent enables improved analysis of nucleated cells with efficient elimination of non-nucleated red cells in an automated instrument by a simple and effective reagent and method.

## Background of the Invention

There are many situations in which analysis of populations of nucleated cells is desirable without interference from the usually very large number of simultaneously present intefering red cells. Analysis of white cells in circulatory blood is an obvious example. Similar problems arise with the analysis of other body fluids, of bone marrow and of tissue biopsy specimens when these are heavily contaminated by red blood cells.

In particular, the examination of the peripheral blood is an important aspect in evaluating the health of the individual. An important parameter within this examination is the differential white cell (or leukocyte) count. This test is presently performed most efficiently by automation of the leukocyte differential using flow cytometry. In this procedure, blood cells in suspension are passed through a transducer or flow cell and the cells are classified on the basis of some measurable parameter such as light absorption, light scatter or electrical impedance. The advantages of these flow cytometry systems compared to microscope-based systems include relatively high sample throughput and the ability to count larger number of cells per sample thereby increasing accuracy and reducing sampling noise. The current instruments are based on either measurement of light scatter or electrical impedance. Single channel light scatter instruments such as ELT 1500® of Ortho Instruments (Westwood, Massachusetts) classify white blood cell subpopulations on the basis of their light scattering characteristics measured at two different angles, typically termed "low" and "orthogonal." Single channel electrical impedance instruments such as the S + series® of Coulter Electronics (Hialeah, Florida) and Cell Dyn 2000® of Unipath Co., Mountain View, California) classify leukocytes based on their volume following exposure to a volume modifying reagent which also lyses the potentially interfering red cells.

Multi channel analyzers, of course, may be employed in various manners to accomplish white cell analysis but, of course, such an approach to analysis is inherently more expensive and usually slower than the use of a single channel. Further, as separate channels are used, there is always the possibility of differences introduced into one channel that are not in the other channels.

The CELL-DYN 3000® single channel white cell analyzer of Unipath Co. performs a conventional five part subpopulation differential based only upon light scattering characteristics of unstained leukocyte cells. Since inherently slow cytochemical staining is not used, the sample throughput of this instrument is kept extremely high. The advances made possible by CELL-DYN 3000® are largely the result of the combination of two innovations, namely measurement of depolarised orthogonal light scatter and with an appropriate sheath reagent such as the reagent of the present invention, enabling better resolution of lymphocytes and monocytes to obtain a five-subpopulation leukocyte differential analysis by use of a single channel.

In commercially available automated instruments using flow cytometry, the leukocyte classification and differentiation is obtained either by light scatter or by electrical impedance related to cell size. In either type of instrument, the red blood- cells in the whole blood sample must be lysed and cleared away prior to the white blood cell determination. In electrical impedance measurement-based systems, quaternary ammonium-salt-based lysing agents have been used. The electrical impedance systems have an adequate response time which substantially nullifies the lytic effect of the lysing agent on the leukocytes. However, this method lyses the white cell membranes, and changes the cellular characteristics of the WBC, the differentiation of leukocyte is obtained only by nuclear size approximation. In light scattering measurement-based systems, the adverse effect of the red blood cell lysing agent upon the light scattering characteristics and the cellular integrity of the white blood cells is more severe, it is biochemical and time dependent and thus more demanding from a design perspective. Lytic agents (used herein interchangeably with "lysing agents") must rapidly lyse red blood cells while simultaneously providing a window in which substantially all of the white blood cell population and their light scattering characteristics are essentially undisturbed from their natural state. This is a simple and efficient one reagent method. Other alternative methods, such as Kim, U.S. Patent No. 4,099,917, describes a method of sensitizing red blood cells with a non-ionic detergent, adding a Formaldehyde fixative, and incubating the blood at 58°C to lyse the red blood cells selectively, leaving leukocytes and platelets for light scatter measurement. This process is rather slow (3 minutes) and complex. Ledis et al., U.S. Patent No. 4,751,179 also describes a complex process of lysing the red blood cells with a lytic diluent followed by fixing the mixture at 60 to 70°C with glutaraldehyde fixing reagent. These multiple processes also require about one minute for a tedious analysis..

A Lytic agent is described in U.S. Serial Number 352,106, filed 5/15/89 to Marshall et al. and assigned to the assignee hereof. A second lytic agent is described in U.S. 4,637,986 issued January 20, 1987 to Brown and assigned to Ortho. Both are for single channel WBC analyzers.

In order to properly count and differentiate white blood cells (WBC), the red blood cells (RBC) first must be lysed and cleared away. At the same time, WBC diluted and exposed in the same leukoprotective sheath reagent must maintain their cellular integrity. For good WBC classification and detection therefore, the sheath system must have several attributes. It must be strong enough to completely lyse normal RBC or indeed, all RBC and at the same time must provide milder and gentle conditions to retain the integrity even of WBC subpopulations which are less robust either because of blood sample aging or weakening in vitro before laboratory analysis or because of pecularities of the blood cells already present in vivo because of disease or therapy. Clearly then, an appropriate balance must be achieved between lysing one type of cell and protecting the other.

On aging, white cells tend to develop vacuoles which exhibit more of a sol like character and are thus less able to withstand hypotonicity, pH stresses and other adverse conditions of the lytic environments. Additionally, in some pathological conditions relatively fresh cells tend to exhibit extreme sensitivity to lytic agents. Further, the interaction of certain anticoagulants with blood cells may create adverse conditions.

Accordingly, it is an object of this invention to substantially lyse red blood cells while protecting substantially all white blood cells including the distinctive characteristics of their subpopulation.

It is a further object of this invention to provide a lytic agent which enables the enumeration of at least five leukocyte subpopulations, identified as neutrophils, lymphocytes, monocytes, eosinophils and basophils, in a single channel flow cytometry, light-scattering system/while allowing an aged and/or weakened blood sample to be analyzed with acceptable fidelity for a longer period of time after sample collection.

It is another object of this invention to provide a lytic agent which has a commercially acceptable shelf life.

These and further objects of the present invention will become apparent to those of skill in the art with reference to the specification and examples.

## Summary of the Invention

The above objects are substantially accomplished in the invention by a more physiologic pH range than the pH above 8 of prior reagents; suitable concentrations of a mild non-ionic detergent; an appropriate tonicity medium and a synergistic combination of selected leukoprotective agents.

The flow cytometry lytic agent of the present invention enables a faithful differential enumeration of circulating nucleated blood cells into subpopulations e.g. nucleated red cells, neutrophils, lymphocytes, monocytes, eosinophils and basophils. It also enables analysis of other flow cytometric tissue prepartions.

It has been found that a single cytoprotector is largely ineffective to completely protect all different types of white blood cells under all handling and clinical conditions. For example, in an article by J. Cross and C.A. Strange, published in Clin. Lab. Haematology, pp. 371 to 375 in 1987, a specific underestimation of WBC in Chronic lymphatic leukemics (CLL) has been explored. Further, white blood cells quickly deteriorate and weaken in normal patient blood samples after they are drawn and kept for more than 12 to 16 hours, especially if they are kept at ambient/room temperature. It is preferred to adjust the tonicity pH and the composition of leukoprotective agents to avoid extreme conditions and to improve speed of analysis, it is preferred not to employ a fixative or an elaborate temperature manipulation. Therefore, a combined erythrolytic leukoprotective reagent system has been found to be essential to enhance the ability of currently used flow cytometry analyzers.

The sheath reagent of the invention is an aqueous solution or dispersion of organic buffer; pH adjusting additives; mild nonionic erythrolytic surfactant; synergistic leukoprotective combination and; selected additives to provide the appropriate tonicity of the reagent.

The organic buffer is preferably triethanolamine or imidazole. An aqueous solution of the buffer and EDTA provide a leukoprotective buffer.

The pH adjusting additives are, for example, triethanolamine and acetic acid.

The mild nonionic surfactant is tween 80, saponin, tween 20 or indeed any appropriate nonionic surfactant or a mixture of these to help lyse RBC and to clear stroma away.

The antimicrobials which also serve as leukoprotectors include phenoxyethanol, phenylethanol, chlorobutanol, chloroxylenol, thimerosal, Nalidixic acid, Nystatin, Streptomycin, Neomycin, Chloramphenicol, Dihydrostreptomycin, propylparaben and other parabens.

Suitable Leukoprotector materials are n butanol, 2-butoxyethanol, 2-ethoxyethanol, 1,3-dimethylurea, Eosin Y, o ethoxybenzoic acid, Erythrosin B and polyvinylalcohols (cold water soluble) as well as those in the following list:.

* Chlorobutanol (1,1,1-Trichloro-2-methyl-2-Propanol)
* Chloroxylenol (4-chloro-3,5-xylenol, 4-chloro-3,5-Dimethylphenol,
* Triethanolamine (2,2',2''-Nitrilotriethanol)
* 2-Ethoxyethanol (Cellosolve, Ethylene glycol monoethyl ether)
* 2-Butoxyethanol (Ethylene glycol monobutyl ether)
* Tween 80 (Polyoxyethylene Sorbitan Monooleate)
* Tween 20 (Polyoxyethylene Sorbitan Monolaurate)
* Eosin Y (C.I 45380, Eosin Yellowish)
* Propyl paraben (p-Hydroxybenzoic Acid propyl ester, n-Propyl, p-Hydroxybenzoate)
* Streptomycin sulfate (Streptomycin sesquisulfate)
* Thimerosal (Sodium ethylmercurithiosalicylate, Merthiolate)
* Ethylene Diamine Tetraacetic Acid (EDTA, [Ethylene dinitrilo] tetraacetic acid)
* Saponins (Sapogenin glycosides from Gypsophila)
* 2-Phenoxyethanol (Ethylene glycol monophenyl ether, Phenyl Cellosolve)
* Nalidixic Acid (1-Ethyl-1,4-dihydro-7-methyl-4-oxo-1,8-naphthyridine-3-carboxylic acid)
* O-Ethoxybenzoic Acid
* Nystatin (Mycostatin)

The method of the present invention provides the lytic agent as described above in combination with a diluted whole blood sample. After the addition of the lytic agent to the diluted whole blood sample, the white blood cell containing sample stream is intersected with a focused laser beam. Four light scattering parameters are then measured at 1-3 degrees, about 10 degrees, and at 90 degrees for total and depolarized light. These measured parameters are thereafter resolved to obtain a five part subpopulation differential enumerating the neutrophils, lymphocytes, monocytes, eosinophils, and basophils. The method of this light scattering analysis is now well known and fully described in operation manuals for the cell DYN 3000 analyzer mentioned above as well as in Serial Number 07/327,416 filed 3/20/89 to De Grooth et al. to be issued on 5/21/91 as U.S. Patent No._____ and incorporated by reference herein.

## Detailed Description of the Invention

A flow cytometry diluent reagent enabling lysis of anucleated red cells with preservation of nucleated cells consisting essentially of an erythrolytically hypotonic aqueous buffer solution of 40 to 200 milliosmoles containing:

(a) at least one $C_1$-$C_{14}$ alkanol;

(b) at least one $C_1$-$C_{20}$ alkoxyalkanol, and

(c) at least one nonionic detergent having an HLB of 14 to 17.

In the preferred embodiment, the anucleate red cell lysing cytoprotective diluent reagent comprises an aqueous solution or dispersion having an osmolality of about 40 to 200 milliosmoles as follows:

|  | Concentration - g/l |
|---|---|
| Triethanolamine/Imidazole | 0.5 - 2.0 |
| EDTA | 0.02 - 0.5 |
| Tween 80/Saponin | 0.05 - 1.0 |
| phenoxyethanol | 0.05 - 1.0 |
| chlorobutanol | 0 - 0.5 |
| n-butanol | 0.5 - 7.0 |
| 2-ethoxyethanol | 0.5 - 7.0 |
| streptomycin | 0.01 - 1.0 |
| polyvinylalcohol | 0.1 - 3.0 |
| dimethylurea | 0 - 1.0 |
| pH | 6.5 - 8.5 |

Whole blood is mixed with an excess of this lytic reagent, typically a 50-fold excess. Lysis of the red blood cells occurs instantly due to the combination of osmotic shock, the action of the non-ionic detergent and pH in the lytic agent. Two other effects are firstly, it functions as a leukoprotective reagent to retard lysis of leukocytes, enabling detection of the subpopulations prior to substantial adverse effects on the white blood cells' optical properties caused by the lytic agent. Secondly, it functions in its more common role as an antimicrobial, allowing the reagent to remain free of microbial contamination for at least one year after preparation. The triethanolamine or imidazole, the buffer commonly used in biochemical solutions, in addition to providing pH buffering capacity, serves to increase the conductivity of the solution so that its presence in the reagent reservoir of the instrument may be readily detected using a pair of electrodes. The Tween 80/or Saponin serves as a red blood cell lytic agent and as a wetting agent, reducing the adhesion of air bubbles as well as blood cells and their debris to the tubing and optical transducer.

Two optimal formulations of the sheath reagent are given below:

|  | g/l | g/l |
|---|---|---|
| triethanolamine | 0.746 | --- |
| Imidazole | --- | 0.476 |
| EDTA | 0.12 | 0.06 |
| Tween 80 | 0.11 | 0.11 |
| Saponin | --- | 0.12 |
| Phenoxyethanol | 0.20 | 0.30 |
| Chlorobutanol | 0.10 | --- |
| n-butanol | 3.00 | 3.00 |
| 2-ethoxyethanol | 3.00 | 3.50 |
| streptomycin sulfate | 0.15 | 0.15 |
| polyvinylalcohol | 0.50 | 0.50 |
| Dimethylurea | --- | 0.20 |
| water | 1000 ml | 1000 ml |
| pH (triethanolamine or acetic acid to adjust) | 8.4 | 7.4 |

In the optimized formulation, a combined leukoprotector is present at a concentration of about 7 g/L, although a useful range of concentrations exists between 4 g/L and 10 g/L. The pH of the buffer may be decreased to pH 6.5 for the Saponin system and 7.8 for the Tween 80 system without significant effects on normal patient blood performance. If the pH is increased above pH 9, partial destruction of white blood cells can occur. The presence of suitable amounts, up to about 1.0 g/L of Tween 80/Saponin, or a similar non-ionic detergent, prevents problems caused by inadequate lysis in specimens typically regarded as difficult to lyse. These include blood controls, whole blood samples from patients with hemoglobinopathies and from those with elevated red blood cell counts. Other critical factors are the tonicity and the combined concentration of leukoprotectors in the system as each characteristic also plays an important role in complete lysing of RBC's.

Other organic buffers can be substituted for imidazole or triethanolamine. Among those with pK at 6.5 to 8.5, boric acid, Tris, glycylglycine and BICENE (available through CalBiochem) can be used in the lytic agent of the present invention. With respect to the non-ionic detergent component of the lytic agent, Saponin, Tween 80® or Tween 20® can be used. Other hydrophilic detergents may be selected from those having polyoxyethylene or saccharide head groups.

A typical automatic blood analyzer is fully described in case Serial No. 352,106 to Marshall et al. and assigned to the assignee hereof. The disclosure of this case is incorporated by reference herein.

The five-part white blood cell differential is determined solely on the basis of light scatter. Since no cytochemical staining or high temperature manipulation and fixation are required, the CELL-DYN 3000® realizes an extremely high throughput (CBC/PLTs/WBC diff at a rate of 109/hour). The lytic agent of the present invention lyses red blood cells instantly, but does not affect the light scattering characteristics of white blood cells within the relevant measurement time. Therefore, cells are characterized and classified under their natural state. Data on the scatter characteristics of each white cell is obtained from four Photodetectors. The "0 degree' detector collects light scattered at a half angle of about 1-3 degrees to the laser beam. Scatter at low angles such as these is mainly a function of cell volume. The '10 degree' detector collects light scattered at a half angle of about 3-10 degrees. Scatter at these angles is strongly affected by gross structural complexity of the cells. Scatter at high angles, such as perpendicular to the laser beam, is also a function of the total amount of structure in the cell and especially: its "fine structuredness." Other things being equal, the higher angle scatter of a cell will increase as, for example, the lobulation of the nucleus increases or as cytoplasmic granulation increases. The CELL-DYN 3000 uses two photomultiplier tubes to collect "90 degree" scatter and "90 degree depolarized" light scatter. This depolarization signal results from multiscattering phenomena within the white blood cells. It is not an artifact of cross depolarization or autofluorescence.

The invention will be more fully illustrated by the Reagents described in the following Table(s).

| Examples | (1) g/l | (2) g/l | (3) g/l | (4) g/l | (5) g/l | (6) g/l | (7) g/l | (8) g/l | (9) g/l | (10) g/l | (11) g/l | (12) g/l | (13) g/l | (14) g/l | (15) g/l |
|---|---|---|---|---|---|---|---|---|---|---|---|---|---|---|---|
| * Triethanolamine | 0.746 | 0.746 | 0.746 | 0.746 | --- | 0.746 | 0.746 | 0.746 | 0.746 | --- | 0.746 | --- | 0.746 | 0.746 | 0.746 |
| * Imidazole | --- | --- | --- | --- | 0.340 | --- | --- | --- | --- | 0.476 | --- | 0.476 | --- | --- | --- |
| * Ethylene Diamine Tetracetic Acid (EDTA) | 0.15 | 0.12 | 0.15 | 0.12 | 0.06 | 0.12 | 0.06 | --- | --- | 0.06 | 0.15 | 0.06 | 0.15 | 0.15 | 0.15 |
| * Acetic Acid to pH | --- | --- | --- | --- | 7.4 | --- | 7.9 | 7.9 | 7.9 | 7.0 | --- | 7.4 | --- | --- | --- |
| * Triethanolamine to pH | 8.4 | 8.4 | 8.4 | 8.4 | --- | 8.4 | --- | --- | --- | --- | 8.2 | --- | 8.2 | 8.2 | 8.2 |
| * Saponin | --- | --- | --- | --- | 0.12 | --- | 0.08 | --- | 0.06 | 0.12 | --- | 0.12 | --- | --- | --- |
| * Tween 80 | 0.11 | 0.11 | 0.11 | 0.11 | 0.11 | 0.11 | --- | 0.11 | --- | 0.11 | 0.12 | 0.11 | 0.12 | 0.11 | 0.11 |
| * Phenoxyethanol | 0.20 | 0.20 | 0.20 | 0.20 | 0.30 | 0.20 | 0.30 | 0.30 | 0.30 | 0.30 | --- | 0.30 | 0.20 | 0.20 | 0.20 |
| * Chlorobutanol | 0.10 | 0.10 | 0.10 | 0.10 | --- | 0.10 | --- | 0.10 | --- | --- | 0.10 | --- | 0.10 | 0.10 | 0.10 |
| * Chloroxylenol | --- | --- | --- | --- | --- | --- | --- | --- | --- | --- | 0.10 | --- | --- | --- | --- |
| * n-Butanol | 3.0 | 3.0 | 3.0 | 3.0 | 3.0 | 3.0 | 3.0 | 3.0 | 3.0 | 3.0 | 1.50 | 3.0 | 3.0 | 3.0 | 3.0 |
| * 2-Butoxyethanol | --- | --- | --- | --- | --- | --- | --- | --- | --- | --- | 1.50 | --- | --- | --- | --- |
| * 2-Ethoxyethanol | 2.5 | 3.0 | 1.5 | 3.5 | 3.5 | 3.0 | 3.0 | 1.5 | 1.5 | 3.5 | 1.5 | 3.5 | 2.0 | 2.5 | 1.5 |
| * 1,3-Dimethylurea | 0.5 | --- | 1.0 | 0.5 | 0.2 | 0.5 | --- | 1.0 | 2.0 | 0.4 | --- | 0.2 | 1.0 | --- | 1.0 |
| * Polyvinyl alcohol | --- | 0.5 | 0.5 | 0.5 | --- | 0.5 | 0.5 | --- | --- | --- | --- | 0.5 | --- | --- | --- |
| * Eosin Y | --- | --- | 0.10 | --- | --- | 0.10 | --- | 0.20 | 0.20 | --- | --- | --- | --- | --- | 0.10 |
| * Nalidixic Acid | --- | --- | --- | --- | --- | --- | --- | --- | --- | --- | --- | --- | 0.10 | --- | --- |
| * O-Ethoxybenzoic Acid | --- | --- | --- | --- | --- | --- | --- | --- | --- | --- | --- | --- | --- | 0.075 | --- |
| * Nystatin | --- | --- | --- | --- | --- | --- | --- | --- | --- | --- | 0.01 | --- | --- | --- | --- |
| * Streptomycin Sulfate | 0.10 | 0.10 | 0.10 | 0.15 | 0.15 | 0.15 | 0.15 | --- | --- | 0.15 | --- | 0.15 | --- | --- | --- |

While certain representative embodiments and details have been shown for the purpose of illustrating the invention, various changes and modifications can be made therein without departing from the scope of the invention as defined in the appended claims.

## Claims

1. A flow cytometry diluent reagent enabling lysis of anucleated red cells with preservation of nucleated cells consisting essentially of an erythrolytically hypotonic aqueous buffer solution of 40 to 200 milliosmoles containing:
   (a) at least one $C_1$-$C_{14}$ alkanol;
   (b) at least one $C_1$-$C_{20}$ alkoxylalkanol, and
   (c) at least one nonionic detergent having an HLB of 14 to 17.

2. A lysing reagent for use in a flow cytometry instrument comprising:
   a. an optimal combined antimicrobial leukoprotector acting as a leukocyte sheath reagent and consisting essentially of:
      1. at least one $C_1$-$C_{14}$ alkanol and,
      2. at least one $C_1$-$C_{20}$ alkoxyalkanol.
   b. an organic buffer with pK of about 6.6 to 8.1, serving to provide a pH buffering capacity and to increase the tonicity and conductivity of the lytic agent; and
   c. a nonionic detergent having an HLB of 14 to 17 to increase the red blood cell lysing capability of the lytic agent.

3. The flow cytometry sheath reagent of Claim 1 wherein said antimicrobial/leukoprotectors are selected from various agents consisting of Phenoxyethanol, Phenylethanol, Chlorobutanol, Chloroxylenol, 1,3-Dimethylurea, Polyvinyl alcohol, Eosin Y, O-Ethoxylbenzoic Acid, thimerosal, nalidixic acid, nystatin, streptomycin, D. hydrostreptomycin chloramphenicol, parabens, and mixtures thereof.

4. The flow cytometry lytic agent of Claim 1 wherein said organic buffer is selected from the group consisting of Triethanolamine/EDTA/Acetic acid, Tris/EDTA/Acetic acid, Imidazole/EDTA/Acitic acid, boric acid, glycylglycine and Bicine.

5. The flow cytometry lytic agent of Claim 1 wherein said nonionic detergent is selected from the group consisting of tween 80, tween 20, saponin, and hydrophilic detergents with polyoxyethylene or saccharide head groups.

6. A flow cytometry lytic agent consisting essentially of an aqueous solution of triethanolamine or imidazole buffer, Tween 80 or Saponin and one ore more of the components defined in claim 1 selected from the group consisting of alkanol, alkoxylalkanol, nonionic detergent or mixtures thereof.

7. A flow cytometry lytic agent consisting essentially of an aqueous solution of triethanolamine or imidazole buffer, Tween 80 or Saponin and the combined antimicrobial leukoprotectors of claim 2.

8. The flow cytometry lytic agent of Claim 5 wherein said a combined antimicrobial/leukoprotectors is present at a total concentration of between 4 g/L and 10 g/L.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | EP-A-0 398 652 (UNILEVER PLC) <br> * page 3, line 19 - line 36 * <br> --- | 1-4 | G01N33/50 <br> //G01N15/14 |
| Y <br><br> D | EP-A-0 136 058 (ORTHO DIAGNOSTICS INC.) <br> * the whole document * <br> & US-A-4 637 986 <br> --- | 1-4 | |
| A | EP-A-0 444 240 (TOA MEDICAL ELECTRONICS CO., LTD.) <br> * page 4, column 5, line 31 - column 6, line 47 * <br> --- | 1-8 | |
| A | EP-A-0 430 750 (ABX) <br> * page 3, line 31 - line 50 * <br> --- | 1-8 | |
| A <br><br> D | WO-A-8 505 684 (COULTER ELECTRONICS, INC.) <br> * the whole document * <br> & US-A-4 751 179 <br> --- | 1,2,5-8 | |
| A | EP-A-0 214 614 (TECHNICON INSTRUMENTS CORPORATION) <br> * the whole document * | 1 | TECHNICAL FIELDS SEARCHED (Int. Cl.5 ) <br><br> G01N |

-----

The present search report has been drawn up for all claims

| Place of search | Date of completion of the search | Examiner |
|---|---|---|
| BERLIN | 26 MARCH 1993 | DE KOK A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)